# EUROPEAN PATENT APPLICATION

(11) **EP 2 123 172 A1**
(43) Date of publication of application: **25.11.2009**
(21) Application number: 07738628.2
(22) Date of filing: 15.03.2007
(51) Int. Cl.: A23K 1/18, A23K 1/16, A61K 36/07, A61P 1/12, A61P 31/04

(54) **METHOD OF PREVENTING DISEASES IN PIGLINGS IN THE WEANING STAGE**

(30) Priority: 28.12.2006 JP 2006354108
(71) Applicant: Yukiguni Maitake Co., Ltd., Minami-Uonuma-shi, Niigata 949-6695 (JP)
(72) Inventor: SHIGA, Akira, Koyu-gun Miyazaki 884-0006 (JP); KAMOSHIDA, Akira, Yokohama-shi Kanagawa 230-0051 (JP); SASA, Yojiro, Minamiuonuma-shi Niigata 949-7302 (JP); TANIGUCHI, Shin, Minamiuonuma-shi Niigata 949-6436 (JP); NISHIBORI, Kozo, Tokamachi-shi Niigata 948-0036 (JP)
(74) Representative: Schwahn, Hartmut
(86) International application number: PCT/JP2007/055178
(87) International publication number: WO 2008/081607

(57) **Abstract**

An object of the present invention is to provide a method for preventing diseases in weaned piglets, which relieves the stress of postweaning piglets and enhances the immunostimulating ability in order to prevent the death due to colibacillosis such as edema disease in postweaning piglets and to increase the rate of raising.

Pig diseases such as colibacillosis, particularly diarrhea, can be prevented by administering a *Grifola*-derived substance selected from one or more of dried *Grifola*, dried *Grifola* powder and a *Grifola* extract to pigs in weaning period, so as to solve the problems of the present invention.

## Description

### TECHNICAL FIELD

The present invention relates to a method for preventing diseases in weaned piglets by using a *Grifola*-derived substance selected from one or more of dried *Grifola*, dried *Grifola* powder and a *Grifola* extract.

More specifically, the present invention relates to a method for preventing diseases in weaned piglets, which prevents colibacillosis, such as edema disease, developed due to a weakened immune system of a weaned piglet and prevents diseases greatly affecting the productivity in pig farming, so as to increase the rate of raising pigs.

### BACKGROUND ART

In pig-farming industry, the productivity of domestic pigs has seriously decreased due to diseases. As competition in producing domestic animals increases, priority has been put on economical and efficient production. As a result, the comfortable environment for feeding pigs has not been provided. Therefore, infection of the intestines, respiratory diseases and the like can easily occur in pigs raised under stressful conditions caused by the change in feed technology and the deterioration of the feeding environment. Particularly in weaned piglets, diseases such as edema disease, porcine reproductive and respiratory syndrome, and postweaning multisystemic wasting syndrome have become a significant issue.

A spate of the pig diseases reduces the farm productivity and gives pig farmers big economic losses. Piglets receive immune antibodies from mother pigs in lactational period so that the risk of infection is low. However, the moved antibodies disappear after weaning and, therefore, diseases can be developed easily. The autoimmunity increases in rearing period so as to reduce the risk of developing diseases. That is, a method for feeding weaned piglets greatly influences the productivity in pig farming.

Conventionally, drugs have been used as measures against diseases. As a result, many drug-resistant bacteria have been developed. On the other hand, in order to avoid ineffectual medications, antibiotics have been used to establish normal flora in the intestines in weaning period. However, by administrating antibiotics, toxins can be released from resistant bacteria proliferated in the intestines to rapidly increase the number of troubles so that pigs may be subjected to over-prescription of medications despite the intentions.

Under these circumstances, it has been required recently for natural products and microorganisms such as lactobacillus to add functionality to a feed or an additive, and materials relating to immunostimulation which greatly influences the productivity have been prospective. For example, a feeding technique of giving a mixed feed provided with a specific herb such as anise or garlic to pigs [JP Patent Publication1] and a feed additive for pigs containing a herb, an yeast, a lactobacillus, etc. [JP Patent Publication2and3] have been proposed.

The inventors of the present application have studied about components contained in the "Maitake" mushroom (*Grifola frondosa*) and the use of the same to discover various effects. For example, the inventors have discovered that a fraction obtained by purifying a *Grifola* hydrothermal extract by an alcohol treatment has an immunostimulating property and an antitumor effect [JP Patent Publication4] and that the fraction usefully effects in influenza virus defense against infection [JP Patent Publication 5], and have filed patent applications. If a substance contained in the "Maitake" mushroom (*Grifola frondosa*) has an immunostimulating property, it can be assumed that the autoimmunity of piglets can be enhanced so as to reduce the risk of developing infection diseases such as colibacillosis.

The inventors of the present application have studied also about application of the "Maitake" mushroom (*Grifola frondosa*) to domestic livestock feeding and have filed a patent application regarding an additive for a livestock feed containing a *Grifola*-derived substance [JP Patent Publication6]. However, the invention relates to a feed additive for domestic animals, which enables production of meat with high safety and good flavor, and is not particularly intended to prevent diseases, such as edema disease of weaned piglets, due to weakened immune systems of pigs.
JP Patent Publication1 :(Kokai) No. 2003-88302 A
JP Patent Publication2: (Kokai) No. 2004-49174 A
JP Patent Publication3: (Kokai) No. 2004-49175 A
JP Patent Publication4: (Kokai) No. 9-238697 A
JP Patent Publication5: (Kokai) No. 2005-145934 A
JP Patent Publication6: (Kokai) No. 2003-259816 A

### DISCLOSURE OF THE INVENTION

### Problems to be Solved by the Invention

An object of the present invention is to provide a method for preventing diseases in weaned piglets, which relieves the stress of postweaning piglets and enhances the immunostimulating ability, so as to prevent the death due to colibacillosis such as edema disease in postweaning piglets and to increase the rate of raising.

### Means for Solving the Problems

The inventors have focused on the "Maitake" mushroom (*Grifola frondosa*) reported as having an immunostimulating property, discovered that the death of weaned piglets due to diarrhea remarkably decreased when dried *Grifola* was given to postweaning piglets, and thus have completed the present invention.

That is, the present invention relates to:
(1) a method for preventing diseases in pigs, wherein a *Grifola*-derived substance selected from one or more of dried *Grifola*, dried *Grifola* powder and a *Grifola* extract is administered to pigs in weaning period;
(2) the method for preventing diseases described in (1), wherein the disease is colibacillosis; and
(3) the method for preventing diseases described in (2), wherein the colibacillosis is diarrhea.

In the present invention, all types of *Grifola* including "Maitake" (*Grifola frondosa*), "Shiromaitake" (*Grifola albicans*), "Choreimaitake" (*Dendropolyporus umbellatus*), "Tonbimaitake" *(Grifola gigantea*) and the like can be used. Recently, artificial cultivation of the fruit bodies of "Maitake" (*Grifola frondosa*) has been made possible. It is preferable to use the fruit bodies of *Grifola* in terms of ensuring a stable supply of raw materials.

Fresh *Grifola,* dried *Grifola* or dried *Grifola* powder can be used. The dried powder is easy to handle and preferable. As dried *Grifola*, any *Grifola* prepared by sun drying, hot-air drying or freeze drying can be used.

Dried *Grifola* can be used as it is, or can also be used in the form of small chips, small pieces or fine pieces after appropriate grinding. It is more general to grind dried *Grifola* into powder using a milling apparatus or the like for use, because this leads to a broader application range. In addition, depending on the feeding situation, *Grifola* powder can be appropriately selected from those with large particle sizes to *Grifola* finer powder with small particle sizes.

Dried *Grifola*, dried *Grifola* powder or a *Grifola* extract can be added independently to a feed. To enable thorough mixing of the *Grifola* added to the feed, dried *Grifola*, dried *Grifola* powder or a *Grifola* extract in the dosage form of powders mixed with an extender and a lubricant, granules, pellets, or the like prepared by general methods can also be added to the feed. When a case of using *Grifola* in the form of powder is explained as an example, an extender and a lubricant are added to the dried *Grifola* powder, and then the resultant is thoroughly mixed. At this time, if necessary, substances other than a *Grifola*-derived substance can be added.

As an extender, lactose, starch or dextrin can be used, for example. As a lubricant, a light liquid paraffin can be used. Particularly, when a *Grifola* extract is used, it is preferable to mix the *Grifola* extract with an extender because the *Grifola* extract becomes easily moistened.

Additionally, a medical agent for preventing diseases can be used together with dried *Grifola,* dried *Grifola* powder or a *Grifola* extract. As a medical agent, a drug for preventing pneumonia or a disease in digestive system can be fed. Also, a microorganism material including lactobacillus having an immunostimulating effect can be used.

It can be assumed that the method for feeding dried *Grifola* according to the present invention to domestic pigs improves immunostimulating ability of the pigs. Therefore, the feed can be administered to not only piglets after weaning but also pigs at any feeding stage with no problem. The method for feeding dried *Grifola* powder according to the present invention provides effects when administered to pigs for a certain period. The feeding period varies depending on the kind of the pig or the feeding stage. However, feeding for at least 30 days can be a rough idea. It is preferable to give the feed continuously during the period.

The feeding amount is preferably around 0.09 to 0.14 g/kg/day.

In the present invention, the weaning period collectively means the weaning preceding stage (about 35 days) and the weaning latter stage (about 35 days).

Additionally, in the present invention, pig diseases include edema disease, PRRS (porcine reproductive and respiratory syndrome) and PMWS (postweaning multisystemic wasting syndrome). Particularly, edema disease is colibacillosis inducing a disease affecting the whole body. Edema disease bacteria can be fixed to and abnormally proliferated in small intestine, so as to induce diseases as well as other E.coli bacteria. Toxins such as verotoxins produced by Edema disease bacteria can be absorbed into the blood to run through the whole body, and the toxins can lead piglets to death.

PRRS induces abortion in late pregnancy or feeding disorders such as increase of dead piglets or lentigo and frail piglets, if infects mother pigs. PRRS also induces interstitial pneumonia in piglets, which shows a respiratory symptom characterized by abdominal breathing like as so-called mystery swin disease.

PMWS represents a disease which increases growth insufficiency of pigs in weaning period to fattening preceding period (5 to 15 weeks old), characterized by decrease in body weight and breathing difficulty, which is sometimes accompanied by an enlarged surface lymph node, dermatitis, anemia, diarrhea, etc.

Pig diarrhea is accelerated by putting stress caused by a rapid change of breast milk to artificial feed, weaning, environmental change, etc., to digestive organs of weanling piglets. Additionally, E.coli bacterium is a great factor to induce diarrhea and degradation in digestive function. E.coli bacteria have cilia to adhere to intestine of a piglet, whereby the bacteria can be proliferated in the intestines to produce toxins in order to induce diarrhea.

### Effects of the Invention

The death of piglets can be prevented and the productivity of pig farming can be increased by giving a *Grifola*-derived substance selected from one or more of dried *Grifola*, dried *Grifola* powder and a *Grifola* extract to postweaning piglets.

This specification includes the contents as disclosed in the description and/or drawings of Japanese Patent Application No. 2006-354108, which is a priority document of the present application.

### BRIEF DESCRIPTION OF THE DRAWINGS

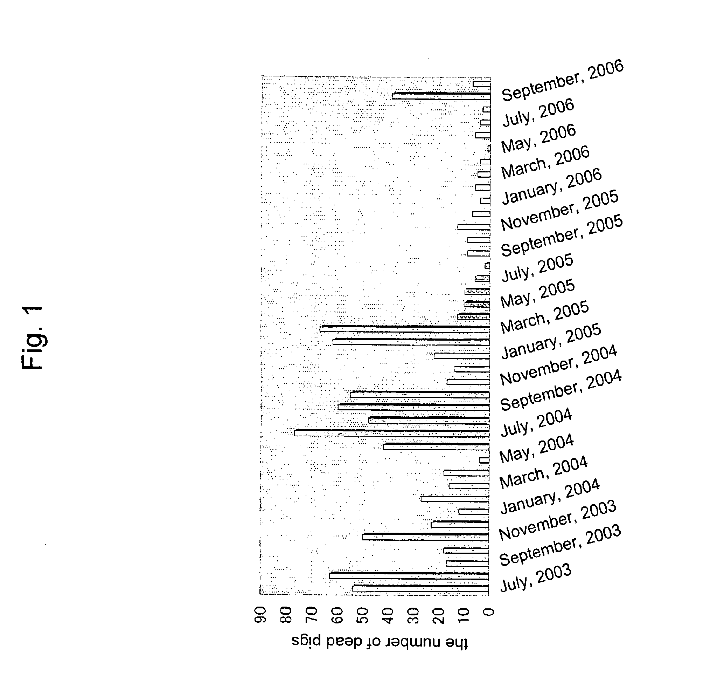

Fig. 1 illustrates the transition of the numbers of dead pigs after weaning.

### BEST MODE FOR CARRYING OUT THE INVENTION

The present invention is described in detail below using Example. However, the present invention is not limited by the Example.

### EXAMPLE 1

### Production of Dried Grifola, Dried Grifola Powder and Grifola Extract

(1) Artificially cultivated fruit bodies of fresh *Grifola frondosa* were lined on the racks of a drying room with racks, and then dried by blowing hot air at approximately 60 °C to approximately 80 °C. By raising the temperature stepwise initially from 60 °C to finally 80 °C for almost one day so as to perform heating and drying, dried fruit bodies of *Grifola frondosa* were obtained. Subsequently, the dried *Grifola frondosa* was powdered with a milling apparatus. On the other hand, a *Grifola* frondosa extract can be produced with water, alcohol, etc., according to general methods.

(2) At the M pig farm, from April to August of 2005, the dried *Grifola frondosa* powder obtained according to the above-described method was fed every day at the ratio of 0.5 % by weight with respect to the weight of the feed to piglets in the weaning period (20 days old) to 50 days old. For the reference group, piglets were fed in the same period of the previous year at the M pig farm without the dried *Grifola* frondosa powder.

Next, the number of dead pigs from diarrhea in alive piglets was checked every month. The results are shown in Table 1 and Fig. 1.

**TABLE 1**

| Month, Year | The number of weaned pigs | The number of dead pigs | Death rate | The number of pigs died from diarrhea | Death rate from diarrhea | |
|---|---|---|---|---|---|---|
| | | | | | (/the number of weaned pigs) | (/the number of dead pigs) |
| January, 2005 | 254 | 22 | 8.7% | 1 | 0.4% | 4.5% |
| February, 2005 | 298 | 62 | 20.8% | 46 | 15.4% | 74.2% |
| March, 2005 | 302 | 67 | 22.2% | 53 | 17.5% | 79.1% |
| April, 2005 | 257 | 13 | 5.1% | 0 | 0.0% | 0.0% |
| May, 2005 | 214 | 10 | 4.7% | 0 | 0.0% | 0.0% |
| June, 2005 | 306 | 10 | 3.3% | 0 | 0.0% | 0.0% |
| July, 2005 | 258 | 6 | 2.3% | 0 | 0.0% | 0.0% |
| August, 2005 | 286 | 2 | 0.7% | 0 | 0.0% | 0.0% |
| September, 2005 | 283 | 9 | 3.2% | 0 | 0.0% | 0.0% |
| October, 2005 | 235 | 9 | 3.8% | 0 | 0.0% | 0.0% |
| Total | 2693 | 210 | 7.8% | 100 | 3.7% | 47.6% |

From the results shown in Table 1, it can be seen that the death rate of piglets after weaning is remarkably reduced by administering the dried *Grifola* frondosa powder from April of 2005, in comparison with the death rate by March of 2005. Additionally, from the results shown in Fig. 1, it can be understood that the death rate of piglets is obviously reduced compared to the number of dead piglets in the previous year, the spread of infection diseases from one pig developing an infection disease to the whole pig farm can be prevented because of the decreased death rate, and that the productivity at the M pig farm is greatly increased after feeding the dried *Grifola frondosa* powder.

In this Example, the dried *Grifola* frondosa powder was used. However, it is obvious that similar results can be obtained using dried *Grifola* or a *Grifola* extract.

All publications, patents and patent applications cited herein are incorporated herein by reference in their entirety.

## Claims

1. A method for preventing diseases in pigs, wherein a *Grifola*-derived substance selected from one or more of dried *Grifola*, dried *Grifola* powder and a *Grifola* extract is administered to pigs in weaning period.

2. The method for preventing diseases according to Claim 1, wherein the disease is colibacillosis.

3. The method for preventing diseases according to Claim 2, wherein the colibacillosis is diarrhea.
